(19) Europäisches Patentamt
European Patent Office
Office européen des brevets

(11) **EP 4 389 033 B1**

(12) **EUROPEAN PATENT SPECIFICATION**

(45) Date of publication and mention
of the grant of the patent:
**17.12.2025  Bulletin 2025/51**

(21) Application number: **23822592.4**

(22) Date of filing: **06.09.2023**

(51) International Patent Classification (IPC):
*A61B 18/00* (2006.01)    *A61B 18/12* (2006.01)
*A61B 18/14* (2006.01)    *A61B 90/00* (2016.01)

(52) Cooperative Patent Classification (CPC):
**A61B 18/12; A61B 18/1206; A61B 18/1492;**
A61B 2018/00351; A61B 2018/00357;
A61B 2018/00577; A61B 2018/00702;
A61B 2018/00791; A61B 2018/00875;
A61B 2090/065

(86) International application number:
**PCT/CN2023/117175**

(87) International publication number:
**WO 2024/093509 (10.05.2024 Gazette 2024/19)**

(54) **READABLE STORAGE MEDIUM, ABLATION SYSTEM, AND ELECTRONIC DEVICE**

LESBARES SPEICHERMEDIUM, ABLATIONSSYSTEM UND ELEKTRONISCHE VORRICHTUNG

SUPPORT DE STOCKAGE LISIBLE, SYSTÈME D'ABLATION ET DISPOSITIF ÉLECTRONIQUE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority:  **03.11.2022  CN 202211368970**

(43) Date of publication of application:
**26.06.2024  Bulletin 2024/26**

(73) Proprietor: **Shanghai Microport Ep Medtech Co.,
Ltd.
Shanghai 201318 (CN)**

(72) Inventors:
• **JIA, Feilong**
**Shanghai 201318 (CN)**
• **SHEN, Lei**
**Shanghai 201318 (CN)**
• **SUN, Tingting**
**Shanghai 201318 (CN)**
• **LIANG, Bo**
**Shanghai 201318 (CN)**
• **SUN, Yiyong**
**Shanghai 201318 (CN)**

(74) Representative: **Patentship
Patentanwaltsgesellschaft mbH
Paul-Gerhardt-Allee 50
81245 München (DE)**

(56) References cited:
WO-A1-2022/028337    CN-A- 111 214 288
CN-A- 112 022 331    CN-A- 113 768 616
CN-A- 113 768 616    US-A1- 2011 066 147
US-A1- 2011 066 147    US-A1- 2011 098 695
US-A1- 2022 313 346

## Description

### TECHNICAL FIELD

The present invention relates to the field of medical devices and, in particular to a readable storage medium, an ablation system and an electronic device.

### BACKGROUND

Pulsed-field ablation (PFA) is an emerging modality for the treatment of atrial fibrillation. Unlike the traditional ablation that injure tissue using heat or cold energy, PFA employs trains of high voltage in very short duration electrical pulses to injure tissue by the mechanism of irreversible electroporation. Studies have shown that the PFA duration and field strength are strongly correlated with electroporation injury of cells. Reversible electroporation of cells will gradually turn to irreversible electroporation as the duration or field strength increases, which will create an ablation lesion in the target tissue. However, in order to achieve a desirable therapeutic effect, the ablation lesion must have an appropriate size. Control of ablation lesion size remains a challenge to be solved.

It is noted the information disclosed in this Background section is merely intended to provide a better understanding of the general context of the present invention and should not be taken as an acknowledgement or any form of admission that the information forms a part of the common general knowledge of those skilled in the art.

US 2011/066147 A1 discloses systems and methods for cardiac ablation treatment. CN 113768616 A discloses an integrated system for cardiac ablation.

### SUMMARY OF THE INVENTION

The invention is defined by the appended independent claims. Further embodiments of the invention are illustrated in the dependent claims. The methods described herein do not form part of the invention, but are useful for understanding the invention.

### SUMMARY OF THE DISCLOSURE

It is an object of the present disclosure to provide a readable storage medium, an ablation system and an electronic device, which can overcome the problem of lacking effective PFA lesion size control associated with the prior art, enhance the success rate of PFA surgery and avoid irreversible thermal damage to tissue.

To this end, the present disclosure provides a readable storage medium having a computer program stored thereon wherein the computer program is executed by a processor to perform the steps of: controlling an ablation catheter to apply pulsed energy to a target tissue and determining electric field energy and a resistance during an ablation process; calculating a theoretical ablation lesion size according to the electric field energy and the resistance; determining whether a difference between the theoretical ablation lesion size and a target ablation lesion size is within a first predetermined range; if so, controlling the ablation catheter to stop applying the pulsed energy to the target tissue; and if not, controlling the ablation catheter to continue applying the pulsed energy to the target tissue, until the difference between the theoretical and target ablation lesion sizes is within the first predetermined range.

The computer program, when executed by the processor, may further perform the step of: determining an effective energy utilization rate of the ablation process.

Calculating the theoretical ablation lesion size according to the electric field energy and the resistance may comprise: calculating the theoretical ablation lesion size according to the electric field energy, the resistance and the effective energy utilization rate.

Optionally, determining the effective energy utilization rate of the ablation process may comprise:

determining a change value of resistance during the ablation process; and determining the effective energy utilization rate according to the change value of resistance and the following equation:

$$C = \frac{R_0 - R_T}{R_0}$$

where $C$ is the effective energy utilization rate, $R_0$ is the resistance at start of the ablation process, $R_T$ is the resistance at time $T$ after start of the ablation process, and $(R_0 - R_T)$ is the change value of resistance.

Optionally, the effective energy utilization rate may be a predetermined value in the range of 0.15-0.35.

Optionally, pressure sensor may be provided at a distal end of the ablation catheter, wherein the pressure sensor is configured to detect a magnitude and a direction of a contact force between the distal end of the ablation catheter and the target tissue.

Determining the effective energy utilization rate of the ablation process may comprise: determining an angle of a fit between the distal end of the ablation catheter and the target tissue according to the direction of the contact force between the distal end of the ablation catheter and the target tissue detected by the pressure sensor; and determining the effective energy utilization rate according to the angle of the fit between the distal end of the ablation catheter and the target tissue, wherein the effective energy utilization rate increases with a decrease in the angle of the fit between the distal end of the ablation catheter and the target tissue.

Optionally, calculating the theoretical ablation lesion size according to the electric field energy, the resistance and the effective energy utilization rate may comprise: calculating the theoretical ablation lesion size according to the electric field energy, the resistance, the effective energy utilization rate and the magnitude of the contact force between the distal end of the ablation catheter and the target tissue.

Optionally, calculating the theoretical ablation lesion size according to the electric field energy, the resistance, the effective energy utilization rate and the magnitude of the contact force between the distal end of the ablation catheter and the target tissue may comprise: calculating a theoretical ablation lesion volume according to the following equation:

$$V(T) = K \cdot C \cdot \int_0^T R_t^{\alpha} \cdot W_t^{\beta} \cdot F_t^{\gamma} dt$$

where $V(T)$ is the theoretical ablation lesion volume at time $T$ after start of the ablation process, $C$ is the effective energy utilization rate, $R_t$ is the resistance at time $T$ after start of the ablation process, $W_t$ is the electric field energy at time $T$ after start of the ablation process, $F_t$ is the magnitude of the contact force between the distal end of the ablation catheter and the target tissue at time $T$ after start of the ablation process, $K$ is a scaling constant, and each of $\alpha$, $\beta$ and $\gamma$ is an exponent not equal to 1.

Optionally, the theoretical ablation lesion size may be the theoretical ablation lesion volume, and the target ablation lesion size may be a target ablation lesion volume.

Optionally, the computer program, when executed by the processor, may further perform the step of: calculating a theoretical ablation lesion depth according to the theoretical ablation lesion volume and the following equation:

$$V = kD^3$$

where $D$ is the theoretical ablation lesion depth, $k$ is a scaling constant, wherein the theoretical ablation lesion size is the theoretical ablation lesion depth, and the target ablation lesion size is a target ablation lesion depth.

Optionally, the computer program, when executed by the processor, may further perform the step of: before the ablation catheter is controlled to apply pulsed energy to the target tissue, determining whether a fit is established between the distal end of the ablation catheter and the target tissue.

Optionally, a pressure sensor may be provided at a distal end of the ablation catheter, wherein the pressure sensor is configured to detect a contact force between the distal end of the ablation catheter and the target tissue, wherein determining whether a fit is established between the distal end of the ablation catheter and the target tissue comprises: determining whether a magnitude of the contact force detected by the pressure sensor is greater than or equal to a first threshold; and if so, determining that the fit has been established between the distal end of the ablation catheter and the target tissue.

Optionally, the computer program, when executed by the processor, may further perform the step of: before the ablation catheter is controlled to apply pulsed energy to the target tissue, determining whether a short is present in a circuit between an energy output device for supplying the pulsed energy to the ablation catheter and the ablation catheter.

Optionally, determining whether a short is present in the circuit between the energy output device for supplying the pulsed energy to the ablation catheter and the ablation catheter may comprise: determining whether the resistance before start of the ablation process exceeds a second threshold; and if not, determining that the short is present in the circuit between the energy output device and the ablation catheter.

Optionally, the computer program, when executed by the processor, may further perform the steps of: before the ablation catheter is controlled to apply the pulsed energy to the target tissue, configuring pulse parameters and calculating a pulse dose according to the pulse parameters; determining whether the pulse dose is within a second predetermined range; and if not, reconfiguring the pulse parameters until the pulse dose is within the second predetermined range.

Optionally, calculating the pulse dose according to the pulse parameters may comprise: calculating the pulse dose according to the following equation:

$$Dose = U^2 \cdot a \cdot d \cdot b \cdot n$$

where *Dose* is the pulse dose, *U* is a pulse voltage, *a* is the number of pulses in each pulse train, *d* is the number of pulse trains delivered in each ablation cycle, *b* is a pulse width, and *n* is the number of ablation cycles.

Optionally, the computer program, when executed by the processor, may further perform the step of: calculating a maximum current limit for the ablation process according to parameter information of electrode leads in the ablation catheter.

Optionally, the computer program, when executed by the processor, may further perform the step of: calculating a minimum resistance limit for the ablation process according to the maximum current limit.

Optionally, the computer program, when executed by the processor, may further perform the step of: determining whether the resistance exceeds the minimum resistance limit in real time during the ablation process.

To the above end, the present disclosure also provides an ablation system comprising an ablation catheter, an energy output device and a controller, each of the ablation catheter and the energy output device communicatively connected to the controller, the energy output device configured to output the pulsed energy to the ablation catheter, the controller comprising the readable storage medium as defined above.

Optionally, a pressure sensor may be provided at a distal end of the ablation catheter, wherein the pressure sensor is configured to detect a magnitude and a direction of a contact force between the distal end of the ablation catheter and a target tissue, wherein the controller is further configured to determine an angle of a fit between the distal end of the ablation catheter and the target tissue according to the direction of the contact force between the distal end of the ablation catheter and the target tissue detected by the pressure sensor; and the ablation system further comprises a display device communicatively connected to the controller, the display device configured to display in real time resistance, the magnitude and direction of the contact force between the distal end of the ablation catheter and target tissue, and an angle of the fit between the distal end of the ablation catheter and the target tissue.

Optionally, a temperature sensor may be further provided at the distal end of the ablation catheter, wherein the temperature sensor is configured to detect an ablation temperature of the target tissue.

To the above end, the present disclosure also provides an electronic device comprising a processor and the readable storage medium as defined above.

The readable storage medium, the ablation system and the electronic device provided in the present disclosure have the following advantages over the prior art:

when the computer program stored in the readable storage medium is executed by the processor, the steps are carried out: controlling the ablation catheter to apply pulsed energy to target tissue and determining electric field energy and resistance during an ablation process; calculating a theoretical ablation lesion size according to the electric field energy and the resistance, and determining whether the difference between theoretical ablation lesion size and a target ablation lesion size is within a first predetermined range; if so, stopping applying pulsed energy to the target tissue; if not, continuing applying pulsed energy to the target tissue, until the difference between theoretical and target ablation lesion sizes is within the first predetermined range. In this way, according to the present invention, the theoretical ablation lesion size is calculated and compared with the target ablation lesion size in real time during the PFA process, thus allowing prediction of PFA efficacy. This can effectively enhance the success rate of the PFA procedure and avoid irreversible thermal damage from being caused to the tissue.

Since the ablation system and electronic device provided in the present disclosure is based on the same inventive concept as the readable storage medium provided in the present invention, they have all the advantages of the readable storage medium. Reference can be made to the above description for more details of the benefits of the ablation system and the electronic device, and further description thereof is omitted here for the sake of brevity.

## BRIEF DESCRIPTION OF THE DRAWINGS

Fig. 1 is a schematic flow diagram of a process carried out when a computer program stored in a readable storage medium is executed according to an embodiment.

Fig. 2 schematically illustrates a measurement performed by the pressure sensor according to an embodiment.

Fig. 3 schematically illustrates decomposition of a force vector according to an embodiment.

Fig. 4 schematically illustrates a force vector and a field-affected region when a horizontal fit is present between a distal end of an ablation catheter and target tissue.

Fig. 5 schematically illustrates a force vector and a field-affected region when a vertical fit is present between a distal end of an ablation catheter and target tissue.

Fig. 6 is a schematic diagram showing the dependence of the size of an ablation lesion on energy of an electric field according to an embodiment.

Fig. 7 schematically illustrates a histogram showing a discrete distribution of experimental effective pulsed energy utilization rate data according to an embodiment.

Fig. 8 is a diagram showing a normal distribution of experimental effective pulsed energy utilization rate data according to an embodiment.

Fig. 9 schematically illustrates a probability-probability (P-P) plot of standardized residual between actual ablation lesion sizes and theoretical ablation lesion sizes calculated according to an equation proposed herein.

Fig. 10 is a schematic flow diagram of a process carried out when a computer program stored in a readable storage medium is executed according to an example.

Fig. 11 is a schematic block diagram illustrating the structure of an ablation system according to an embodiment.

Fig. 12 is a schematic block diagram illustrating the structure of an electronic device according to an embodiment.

In figures,
100, an ablation catheter; 110, a head electrode; 120, a ring electrode; 200, an energy output device; 300, a controller; 400, a display device; 510, a processor; 520, a communication interface; 530, a readable storage medium; and 540, a communication bus.

## DETAILED DESCRIPTION

Readable storage media, ablation systems and electronic devices proposed herein will be described below with respect to particular embodiments and with reference to the accompanying drawings. From the following description, advantages and features of the present invention will become more apparent. It is noted that the figures are provided in a very simplified form not necessarily drawn to exact scale and for the only purpose of facilitating easy and clear description of the embodiments. In order that the objects, features and advantages of the present invention can be more apparent and readily understood, reference is to be made to the accompanying drawings. It would be recognized that architectural, proportional, dimensional and other details in the figures are presented only for the purpose of facilitating, in conjunction with the disclosure herein, the understanding and reading of those familiar with the art, rather than being intended to limit conditions under which the present invention can be implemented. Therefore, any and all architectural modifications, proportional variations or dimensional changes that achieve the same or similar benefits and objects as the present invention are considered to fall within the scope of the teachings herein.
It is noted that, as used herein, relational terms such as first and second, etc., are only used to distinguish one entity or operation from another entity or operation, and do not necessarily require or imply these entities having such an order or sequence. Moreover, the terms "comprise," "include," or any other variations thereof are intended to cover a non-exclusive inclusion within a process, method, article, or apparatus that comprises a list of elements including not only those elements but also those that are not explicitly listed, or other elements that are inherent to such processes, methods, goods, or equipment. In the case of no more limitation, the element defined by the sentence "includes a ..." does not exclude the existence of another identical element in the process, the method, or the device including the element.
Reference throughout this specification to "one embodiment", "some embodiments", "an example", "a specific example" "some examples" or the like means that a particular feature, structure, material, or characteristic described in connection with the embodiments or examples is included in at least one embodiment or example of the invention. Thus, the appearances of those phrases in various places throughout this specification are not necessarily referring to the same embodiment or example of the invention.
In principle, the present invention seeks to provide a readable storage medium, an ablation system and an electronic device, which can overcome the problem of lacking effective pulsed-field ablation (PFA) lesion size control associated with the prior art, enhance the success rate of PFA surgery and avoid irreversible thermal damage to tissue.
It is noted that the readable storage medium provided in the present invention is applicable to the ablation system and electronic device provided in this invention. The electronic device provided in the present invention may be a personal computer, a mobile terminal or the like. The mobile terminal may be a hardware device running any of various operating systems (OS), such as a mobile phone or a tablet computer. In addition, notably, as would be appreciated by those skilled in the art, references herein to "plurality" are meant to include two. As used herein, the term "distal end" refers to an end away from an operator (i.e., closer to target tissue), while "proximal end" refers to an end closer to the operator (i.e., away from the target tissue).

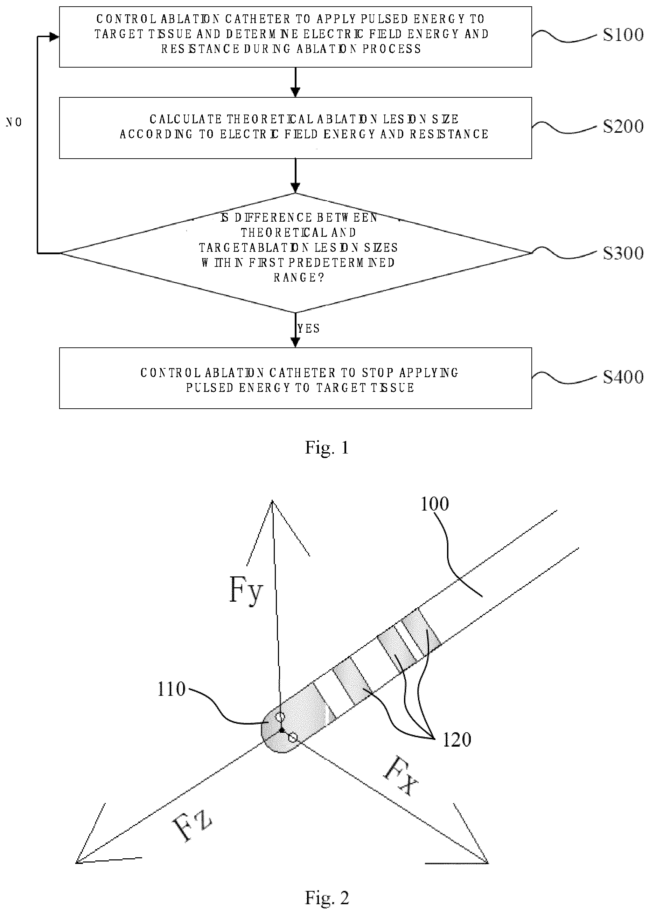

To achieve the above goal, the present disclosure provides a readable storage medium storing therein a computer program. Fig. 1 is a schematic flow diagram of a process carried out when the computer program stored in the readable storage medium is executed according to an embodiment of the present invention. As shown in Fig. 1, when the computer program stored in the readable storage medium is executed, steps S100 to S400 below are performed.

Step S100: Control an ablation catheter to apply pulsed energy to target tissue and determine electric field energy and resistance during the ablation process.

Step S200: Calculate a theoretical ablation lesion size based on the electric field energy and resistance.

Step S300: Determine whether the difference of theoretical ablation lesion size and a target ablation lesion size is within the first predetermined range.

If yes, control proceeds to step S400; if no, control loops back to step S100.

Step S400: Control the ablation catheter to stop applying pulsed energy to the target tissue.

In this way, according to the present disclosure, the theoretical ablation lesion size is calculated and compared with the target ablation lesion size in real time during the PFA process, thus allowing prediction of PFA efficacy. This can enhance the success rate of the PFA procedure and avoid irreversible thermal damage from being caused to the tissue. Notably, as would be appreciated by those skilled in the art, the first predetermined range may be set as actually required by the ablation procedure, and the present invention is not limited in this regard.

In particular, the ablation may be performed in either of monopolar and bipolar modes. Monopolar RFA may involve energizing a head electrode 110 (see Fig. 2) and at least one reference electrode to enable them to receive electrical pulses. Bipolar RFA may involve energizing a head electrode 110 and at least one ring electrode 120 to enable them to receive electrical pulses. The head electrode 110 and the at least one ring electrode 120 (see Fig. 2) may be opposite in polarity. It will be understood that, in case of bipolar ablation, each of the electrodes may be selectively positive or negative. That is, it is not the case that either the head electrode 110 or any ring electrode 120 always serves as a negative or positive electrode, and there may be one or more negative electrodes, or one or more positive electrodes. Notably, as would be appreciated by those skilled in the art, when in the monopolar mode, the term "resistance", as used herein, refers to resistance between the energized head electrode 110 and reference electrode; and when in the bipolar mode, the term "resistance", as used herein, refers to resistance between the energized head electrode 110 and ring electrode 120.

Notably, as would be appreciated by those skilled in the art, the readable storage medium provided in the present invention may be implemented as one of various computer-readable media, or any combination thereof. Each of the readable media may be a computer-readable signal medium or a computer-readable storage medium. The computer-readable storage medium may be, for example, but is not limited to, an electronic, magnetic, optical, electromagnetic, infrared, or semiconductor system, apparatus, or device, or any suitable combination of the foregoing. More specific examples (a non-exhaustive list) of the computer-readable storage medium would include the following: an electrical connection comprising one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. As used herein, the phase "computer-readable storage medium" may refer to any tangible medium that can contain, or store a program for use by or in connection with an instruction execution system, apparatus, or device.

The computer-readable signal medium may include a propagated data signal with computer-readable program code embodied therein, for example, in baseband or as a part of a carrier wave. Such a propagated signal may take any of a variety of forms, including, but not limited to, electro-magnetic, optical, or any suitable combination thereof. The computer-readable signal medium may also be any computer-readable medium that is not a computer-readable storage medium and that can communicate, propagate, or transport a program for use by or in connection with an instruction execution system, apparatus, or device. Program code embodied on the computer readable medium may be transmitted using any appropriate medium, including, but not limited to, wireless, electric wire, optical fiber cable, RF, etc., or any suitable combination of the foregoing.

In an exemplary embodiment, the computer program, when executed by a processor, may further perform the step of obtaining an effective energy utilization rate during the ablation process.

Accordingly, the calculation of theoretical ablation lesion size based on the electric field energy and resistance may include: calculating theoretical ablation lesion size based on the electric field energy, the resistance and the effective energy utilization rate.

In practice, the pulsed energy applied by the ablation catheter does not all act on the target tissue. That is, in practice, the pulsed energy applied in the ablation process will be partially lost. Accordingly, introducing the effective energy utilization rate to the calculation of theoretical ablation lesion size according to this embodiment can ensure that the calculated theoretical ablation lesion size is more accurate and allows even better prediction of PFA efficacy, which can more effectively enhance the success rate of the ablation procedure and more effectively avoid thermal damage to the tissue.

The ablation catheter may be provided at a distal end thereof with a pressure sensor for detecting the magnitude and direction of a contact force between the distal end of the ablation catheter and the target tissue. The pressure sensor can accurately detect both the magnitude and direction of the contact force between the distal end of the ablation catheter and the target tissue. This allows the operator to achieve precision ablation in practice by controlling snugness of a fit between the distal end of the ablation catheter 100 and the target tissue (which depends on the magnitude of the contact force) and an angle of the fit (which depends on an angle between the direction of the contact force and an axis of the distal end of the ablation catheter 100).

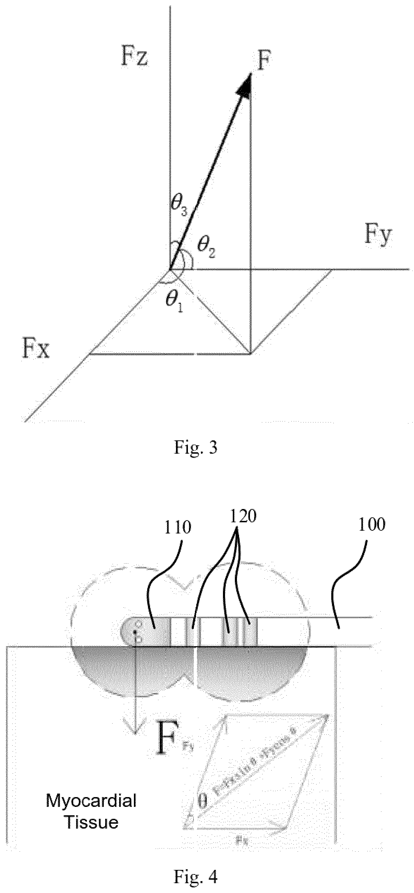

Reference is now made to Figs. 2 and 3. Fig. 2 schematically illustrates a measurement performed by the pressure sensor according to an embodiment of the present invention. Fig. 3 schematically illustrates resolution of a force vector according to an embodiment of the present invention. As shown in Figs. 2 and 3, the pressure sensor can detect components $F_x$, $F_y$ and $F_z$ of the contact force $F$ between the distal end of the ablation catheter 100 and the target tissue along the X, Y and Z axes, respectively, and angles $\theta_1$, $\theta_2$ and $\theta_3$ of the contact force $F$ between the distal end of the ablation catheter 100 and the target tissue and the X, Y and Z axes, respectively. In particular, the contact force $F$ between the distal end of the ablation catheter 100 and the target tissue may be calculated according to the following equation:

$$F = F_x \cos\theta_1 + F_y \cos\theta_2 + F_z \cos\theta_3.$$

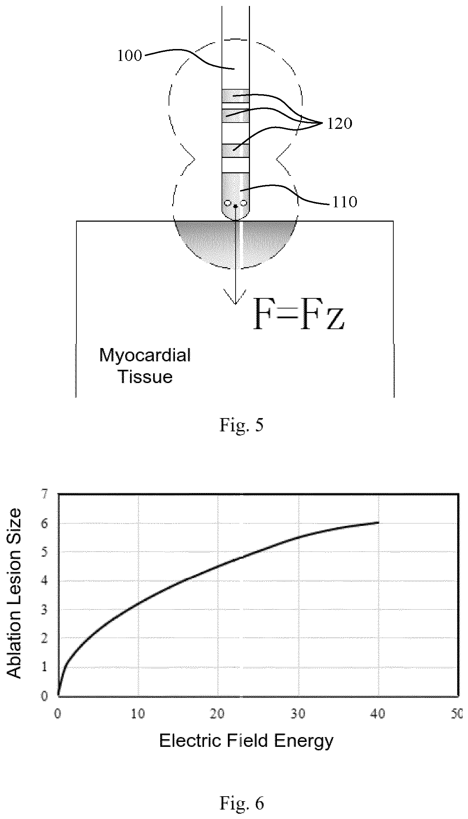

Reference is now to Figs. 4 and 5. Fig. 4 schematically illustrates a force vector and a field-affected region when a horizontal fit is established between the distal end of the ablation catheter 100 and the target tissue. Fig. 5 schematically illustrates a force vector and a field-affected region when a vertical fit is established between the distal end of the ablation catheter 100 and the target tissue. In Fig. 5, the area denoted by the dotted line represents a region affected by the electric field energy, and the gray-shaded area represents a field-affected myocardial region (i.e., the target tissue). As shown in Figs. 4 and 5, in case of a horizontal fit being established between the distal end of the ablation catheter 100 and the target tissue (i.e., an angle of the fit is 0°), the contact force between the distal end of the ablation catheter 100 and the target tissue is oriented perpendicular to the axis of the distal end of the ablation catheter 100 (i.e., the axis of the distal end of the ablation catheter 100 is oriented at an angle of 90° with respect to the contact force F). As a result, both the head electrode 110 and the ring electrode 120 of the ablation catheter 100 are brought into contact with the target tissue, leading to a relatively large field-affected region of the pulsed energy. In case of a vertical fit being established between the distal end of the ablation catheter 100 and the target tissue (i.e., an angle of the fit is 90°), the contact force between the distal end of the ablation catheter 100 and the target tissue is oriented collinear with the axis of the distal end of the ablation catheter 100 (i.e., the axis of the distal end of the ablation catheter 100 is oriented at an angle of 180° with respect to the contact force F). As a result, only the head electrode 110 is brought into contact with the target tissue, while the ring electrode 120 is out of contact with the target tissue, leading to a relatively small field-affected region of the pulsed energy. In this way, precision ablation can be achieved in practice by controlling snugness of a fit between the distal end of the ablation catheter 100 and the target tissue (which depends on the magnitude of the contact force) and an angle of the fit (which depends on the direction of the contact force). Notably, as would be appreciated by those skilled in the art, the angle of the fit between the distal end of the ablation catheter 100 and the target tissue is equal to the angle between the axis of the distal end of the ablation catheter 100 and the direction of the contact force minus 90°.

In an exemplary embodiment, the calculation of theoretical ablation lesion size based on the electric field energy, the resistance and the effective energy utilization rate may include: calculating theoretical ablation lesion size based on the electric field energy, the resistance, the effective energy utilization rate and the contact force between the distal end of the ablation catheter and the target tissue. In particular, a theoretical ablation lesion volume may be calculated according to Eqn. (1) below:

$$V(T) = K \cdot C \cdot \int_0^T R_t^\alpha \cdot W_t^\beta \cdot F_t^\gamma \, dt \qquad\qquad (1)$$

where $V(T)$ is the theoretical ablation lesion volume at time $T$ after the start of ablation, $C$ is the effective energy utilization rate, $R_t$ is the resistance at time $t$ after the start of ablation; $W_t$ is the electric field energy at time $t$ after the start of ablation, $F_t$ is the contact force between the distal end of the ablation catheter and the target tissue at time $t$ after the start of ablation, $K$ is a scaling constant, and $\alpha$, $\beta$ and $\gamma$ are exponents not equal to 1.

For ease of understanding, detail description of Eqn. (1) is set forth below. Specifically, the resulting ablation lesion depends on the electric field strength, the resistance, the contact force between the distal end of the ablation catheter 100 and the target tissue and the duration of ablation. Given this fact, the inventors propose herein that the volume $V$ of the ablation lesion is proportional to the product of the resistance $R$ after ablation, the electric field energy $W,$ the contact force $F$ and the duration of ablation $t$, i.e., Eqn. (2) below:

$$V = K \cdot C \cdot R \cdot W \cdot F \cdot t \qquad (2)$$

where $C$ denotes the effective energy utilization rate.

The electric field energy $W$ can be calculated according to Equations (3) and (4) below:

$$W = \frac{1}{2} \varepsilon \int_\tau E^2 d\tau = \frac{1}{2} \varepsilon E^2 \tau \qquad (3)$$

$$E = \int_\tau \frac{3Q}{4\pi \varepsilon r^3} d\tau \, / \, 4\pi r^2 = \frac{Q}{4\pi \varepsilon r^2} \qquad (4)$$

where $\tau$ is the volume of a space of the electric field, $E$ is strength of the field, $Q$ is an amount of electric charge, $\varepsilon$ is the dielectric constant and $r$ is a radius of the space of the electric field.

When the electrode at the distal end of the ablation catheter 100 is approximately spherical, the equation expressing the energy $W$ of the electric field created by the distal electrode (i.e., Eqn. (3)) can be simplified as Eqn. (5) below:

$$W = \frac{Q^2}{24\pi \varepsilon r} \qquad (5)$$

Notably, as would be appreciated by those skilled in the art, since the shape and size of the space of the electric field created by the distal electrode depends on the shape and size of the distal electrode, the volume $\tau$ and the radius $r$ of the space of the electric field can be determined from the shape and size of the distal electrode.

Eqn. (2) defines the ablation lesion size as a linear function of a plurality of factors including the effective utilization $C$ of pulsed energy, the resistance after ablation $R$, the electric field energy $W$ and the contact force $F$. However, in practice, the dependence of the ablation lesion size $V$ on the resistance after ablation $R$, the electric field energy $W$ and the contact force $F$ is nonlinear. In particular, reference is made to Fig. 6, a schematic diagram showing the dependence of the ablation lesion size on the electric field energy according to an embodiment of the present invention. As shown in Fig. 6, the ablation lesion size varies as an exponential function of the electric field energy, the greater the electric field strength, the larger the ablation lesion size. Similarly, the ablation lesion size also varies as an exponential function of the contact force between the distal end of the ablation catheter 100 and the target tissue, the greater the contact force, the larger the ablation lesion size. Similarly, the ablation lesion size varies as an exponential function of the resistance, while the smaller the resistance, the larger the ablation lesion size based on these, the exponents $\alpha$, $\beta$ and $\gamma$ are introduced to optimize Eqn. (2) into Eqn. (6) below:

$$V = K \cdot C \cdot R^\alpha \cdot W^\beta \cdot F^\gamma \cdot t \qquad (6)$$

In addition, when the distal electrode of the ablation catheter 100 is approximately spherical, Eqn. (7) below can be obtained from $Q = \dfrac{U}{R} \cdot t$ (an equation expressing the amount of electric charge), and Equations (5) and (6):

$$V = K \cdot C \cdot R^\alpha \cdot \left(\frac{UIt^2}{24\pi \varepsilon r R}\right)^\beta \cdot F^\gamma \cdot t \qquad (7)$$

where $I$ represents an ablation current.

As the electric field energy and the contact force may vary during ablation, in order to additionally reduce the error between theoretical and actual ablation lesion sizes, Eqn. (6) may be further optimized using an integration technique, thereby obtaining Eqn. (1) above proposed herein, which expresses theoretical ablation lesion size.

Notably, as would be appreciated by those skilled in the art, $.\alpha$, $,\beta$ and $\gamma$ are exponents not equal to 1, and $K$, $\alpha$, $\beta$ and $\gamma$ may be empirically determined, in particular by performing principal components analysis and weight analysis on experimental data.

In a first embodiment, obtaining the effective energy utilization rate of the ablation process includes: obtaining a change value of resistance over the ablation process; and obtaining the effective energy utilization rate based on the change value of resistance and Eqn. (8) below:

$$C = \frac{R_0 - R_T}{R_0} \tag{8}$$

where $C$ denotes the effective energy utilization rate, $R_0$ represents resistance before the start of ablation, and $R_T$ is resistance at time T after the start of ablation. Thus, the change value of resistance is $(R_0 - R_T)$.

As can be seen from Eqn. (8), the effective energy utilization rate $C$ obtained according to the first embodiment dynamically varies with the progress of the ablation process. Since the effective energy utilization rate $C$ is calculated based on an resistance profile over the ablation process according to the first embodiment, it is more realistic and allows theoretical ablation lesion size calculated based thereon to be more accurate and closer to the actual ablation lesion size, thereby enabling more precise PFA efficacy prediction.

In particular, Eqn. (8) may be derived from Eqn. (9) below:

$$C = \frac{W_{effective}}{W_{total}} = \frac{Q^2_{effective}}{Q^2_{total}} = \frac{I^2 \Delta R t}{U I t} = \frac{\Delta R}{R_0} \tag{9}$$

where $\Delta R = R_0 - R_T$.

Moreover, Eqn. (10) below can be obtained by substituting Eqn. (8) into Eqn. (1):

$$V(T) = K \cdot \frac{R_0 - R_T}{R_0} \cdot \int_0^T R_t^{\alpha} \cdot W_t^{\beta} \cdot F_t^{\gamma} dt \tag{10}$$

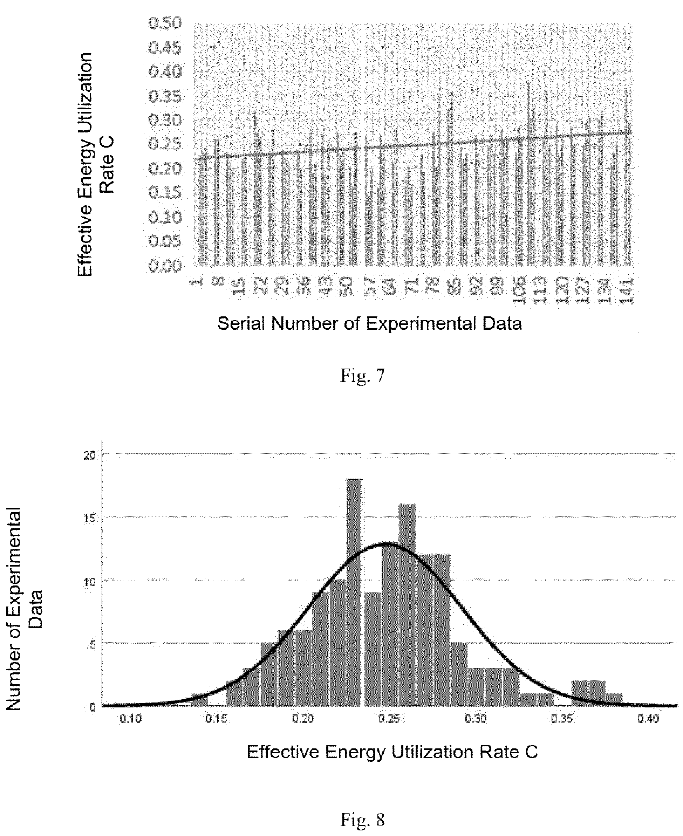

In a second embodiment, the effective energy utilization rate is a predetermined value in the range of 0.15-0.35. In particular, reference is now made to Figs. 7 and 8. Fig. 7 schematically illustrates a histogram showing a discrete distribution of experimental effective pulsed energy utilization rate data according to an embodiment of the present invention. Fig. 8 schematically illustrates a normal distribution of effective pulsed energy utilization rate data according to an embodiment of the present invention. As shown in Figs. 7 and 8, massive experimental data have shown that an effective energy utilization rate $C$ of PFA processes is about 0.15-0.35. Accordingly, in the present disclosure, the effective energy utilization rate is set to a value selected from the range of 0.15-0.35. This can also ensure that the calculated theoretical ablation lesion size is close to the actual ablation lesion size.

Further, as can be seen from the above description in connection with Figs. 4 and 5, in case of a horizontal fit being established between the distal end of the ablation catheter 100 and the target tissue (i.e., an angle of the fit is 0°), both the head electrode 110 and the ring electrode 120 of the ablation catheter 100 are brought into contact with the target tissue, leading to a relative large field-affected region of the pulsed energy. Moreover, when a vertical fit is established between the distal end of the ablation catheter 100 and the target tissue (i.e., an angle of the fit is 90°), only the head electrode 110 is brought into contact with the target tissue, while the ring electrode 120 is out of contact with the target tissue, leading to a relatively small field-affected region of the pulsed energy. Therefore, the angle of the fit between the distal end of the ablation catheter 100 and the target tissue also has an impact on the effective energy utilization rate. Accordingly, in a third embodiment hereof, a method of obtaining the effective energy utilization rate of the PFA process is proposed.

Specifically, the method proposed in the third embodiment includes: obtaining the angle of the fit between the distal end of the ablation catheter and the target tissue based on the contact force between the distal end of the ablation catheter and the target tissue detected by the pressure sensor; and determining the effective energy utilization rate based on the angle of the sung fit between the distal end of the ablation catheter and the target tissue, wherein the effective energy utilization rate increases with a decrease in the angle of the sung fit between the distal end of the ablation catheter and the target tissue.

EP 4 389 033 B1

Notably, as would be appreciated by those skilled in the art, in practice, a mapping between the angle of the fit and the effective energy utilization rate may be established from a lot of experimental data, according to which, the effective energy utilization rate may be determined from the actual angle of the fit. Such mappings between the angle of the fit and the effective energy utilization for various ablation locations and modalities may be defined in the computer program, and the present invention is not limited in this regard.

In an exemplary embodiment, when the computer program is executed by the processor, the step of calculating a theoretical ablation lesion depth from the theoretical ablation lesion volume and according to Eqn. (11) below may be carried out:

$$V = kD^3 \qquad\qquad (11)$$

where $D$ is theoretical ablation lesion depth, and $k$ is scaling constant and the specific value of $k$ may be determined through an experimentation. Thus, after the volume $V$ of theoretical ablation lesion is calculated according to Eqn. (1), the depth $D$ of theoretical ablation lesion can be in turn calculated according to Eqn. (11).

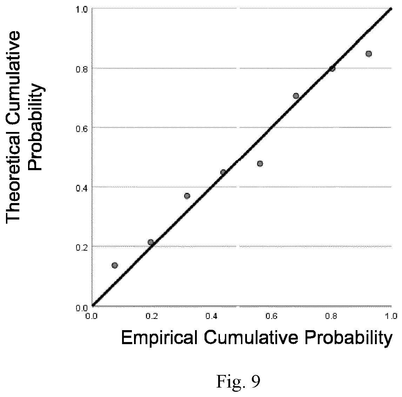

Fig. 9 schematically illustrates a probability-probability (P-P) plot of standardized residual between theoretical ablation lesion sizes calculated according to the equation proposed herein and actual ablation lesion sizes, in which theoretical cumulative probabilities obtained from theoretical ablation lesion sizes and the empirical cumulative probabilities obtained from the actual ablation lesion sizes. As shown in Fig. 9, theoretical ablation lesion sizes calculated according to Eqn. (1) show very small deviations from the actual ablation lesion sizes. That is, theoretical ablation lesion sizes are very close to the actual ablation lesion sizes. Therefore, the readable storage medium provided in the present invention can achieve effective ablation lesion size control, which can enhance the success rate of an ablation procedure and avoid irreversible thermal damage to tissue. Notably, as would be appreciated by those skilled in the art, the P-P plot of standardized residual may be plotted from theoretical and actual ablation lesion sizes using any suitable conventional technique, and further description thereof is omitted herein.

In an exemplary embodiment, when the computer program is executed by the processor, the step of determining whether a fit has been established between the distal end of the ablation catheter 100 and the target tissue may be performed, before the pulsed energy is applied by the ablation catheter 100 to the target tissue.

In this way, ablation is performed only when contact has been confirmed between the distal end of the ablation catheter 100 (see Fig. 2) and the target tissue. This can maximize delivery of the pulsed energy (e.g., pulsed electric field energy) to the target tissue and minimize delivery thereof to the blood, thereby enabling even better ablation efficacy.

Further, determining whether a fit has been established between the distal end of the ablation catheter 100 and the target tissue may include: determining whether the magnitude of the contact force detected by the pressure sensor is greater than or equal to a first threshold. If so, it is determined that a fit has been established between the distal end of the ablation catheter 100 and the target tissue.

Specifically, if the magnitude of the contact force detected by the pressure sensor is greater than or equal to the first threshold, it is indicated that a fit has been established between the distal end of the ablation catheter 100 and the target tissue. Otherwise, if the magnitude of the contact force detected by the pressure sensor is less than the first threshold (e.g., 5 gf), it is indicated that a fit has not been established yet between the distal end of the ablation catheter 100 and the target tissue. In the latter case, the operator may attempt to establish a fit between the distal end of the ablation catheter 100 and the target tissue through adjusting a posture of the distal end of the ablation catheter 100. Notably, as would be appreciated by those skilled in the art, the first threshold may be set as actually required by the ablation procedure, and the present invention is not limited in this regard.

Additionally, as would be appreciated by those skilled in the art, the contact force between the distal end of the ablation catheter 100 and the target tissue may be monitored in real time, in order to effectively avoid safety issues that may arise from an excessive level of the contact force between the distal end of the ablation catheter 100 and the target tissue.

In an exemplary embodiment, when the computer program is executed by the processor, the step of determining whether there is a short in a circuit between an energy output device 200 for supplying pulsed energy to the ablation catheter 100 and the ablation catheter 100 may be performed, before the pulsed energy is applied by the ablation catheter 100 to the target tissue.

As pulsed energy cannot be supplied to the ablation catheter 100 if there is a short in the circuit between the energy output device 200 and the ablation catheter 100, ablation may be carried out only when it has been confirmed that the circuit between the energy output device 200 and the ablation catheter 100 is operating normally (i.e., there is no short circuit or another condition preventing normal discharge therein). This can additionally ensure effectiveness of the ablation procedure. Notably, as would be appreciated by those skilled in the art, when a short is identified in the circuit between the energy output device 200 and the ablation catheter 100, troubleshooting may be performed to find out the cause of the short circuit, and a countermeasure may be taken to remedy the fault to regain normal operation of the circuit between the energy output device 200 and the ablation catheter 100, thereby enabling the ablation catheter 100 to correctly perform ablation with the aid of the energy output device 200.

Further, determining whether there is a short in the circuit between the energy output device 200 for supplying pulsed energy to the ablation catheter 100 and the ablation catheter 100 may include: determining whether the resistance before the start of ablation is greater than a second threshold. If not, it is determined that there is a short in the circuit between the energy output device 200 and the ablation catheter 100. Specifically, when a short occurs in the circuit between the energy output device 200 and the ablation catheter 100 before the start of ablation, the resistance between the head electrode 110 of the ablation catheter 100 and a counter electrode is relatively small. On the contrary, if the circuit between the energy output device 200 and the ablation catheter 100 operates normally before the start of ablation, the resistance between the head electrode 110 of the ablation catheter 100 and the counter electrode is relatively large. A short in the circuit between the energy output device 200 and the ablation catheter 100 can be accurately identified by comparing the resistance before the start of ablation with the second threshold. Notably, as would be appreciated by those skilled in the art, in the monopolar mode, the counter electrode refers to the aforementioned reference electrode. However, in the bipolar mode, the counter electrode refers to the aforementioned ring electrode 120. Notably, as would be also appreciated by those skilled in the art, the second threshold may be set as desired, for example, to 60 ohms, and the present disclosure is not limited to any particular value thereof.

In an exemplary embodiment, when the computer program is executed by the processor, the following steps may be carried out: before the ablation catheter 100 is controlled to apply the pulsed energy to the target tissue, configuring pulse parameters and calculating a pulse dose according to the pulse parameters; determining whether the pulse dose is within a second predetermined range; if not, reconfiguring the pulse parameters, until the pulse dose is within the second predetermined range. In this way, before the start of ablation, the pulse parameter can be set to an appropriate value, which can thus ensure subsequent creation of a sufficiently sized ablation lesion within the target tissue. Notably, as would be appreciated by those skilled in the art, the second predetermined range may be determined as required by the target tissue. For example, when the target tissue is myocardial, the second predetermined range may be determined to be from 800 $V^2 \cdot s$ to 2000 $V^2 \cdot s$.

Further, calculating the pulse dose according to the pulse parameter may include: calculating the pulse dose according to Eqn. (12) below:

$$Dose = U^2 \cdot a \cdot d \cdot b \cdot n \qquad (12)$$

where *Dose* is the pulse dose, *U* is the pulse voltage, *a* is the number of pulses in each pulse train, *d* is the number of pulse trains delivered in each ablation cycle, *b* is a pulse width (the width of each pulse), and *n* is the required number of ablation cycles.

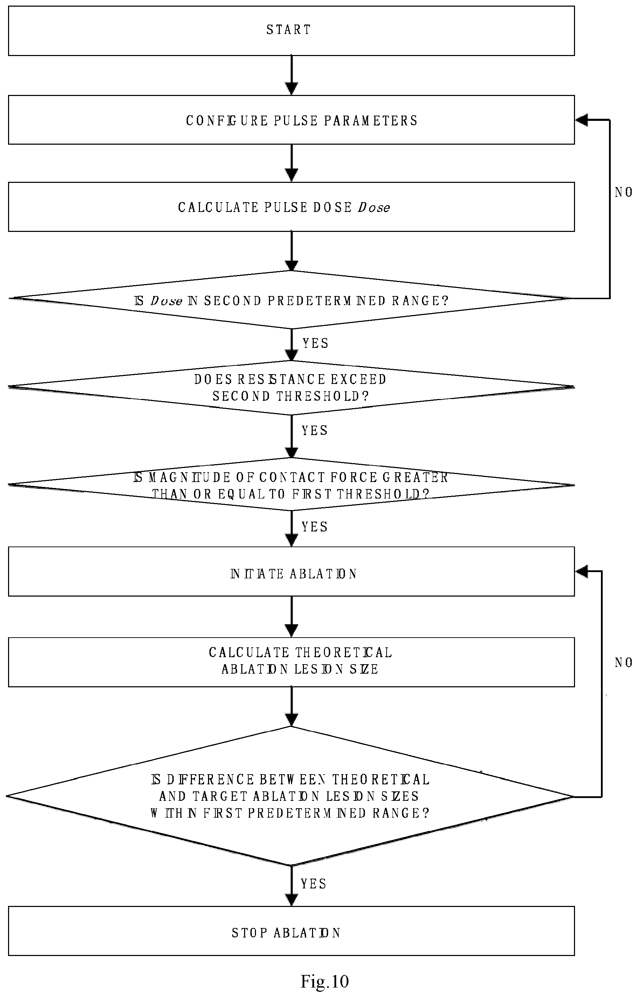

Fig. 10 is a schematic flow diagram of a process carried out when the computer program stored in the readable storage medium is executed according to an example of the present invention. As shown in Fig. 10, before the start of PFA, the pulse parameters are configured, and the pulse dose *Dose* is then calculated according to the configured pulse parameters. If the calculated pulse dose lies within the second predetermined range (e.g., 800 $V^2 \cdot s$ to 2000 $V^2 \cdot s$), it is then determined whether the resistance is greater than the second threshold (e.g., 60 ohms). If the resistance is greater than the second threshold (e.g., 60 ohms), it is then determined whether the contact force is greater than or equal to the first threshold (e.g., 5 gf). If yes, ablation is performed, and during the ablation process, theoretical ablation lesion size is calculated in real time and compared with the target ablation lesion size. If the difference between theoretical and target ablation lesion sizes lies within the first predetermined range, the ablation process is stopped. Otherwise, the ablation process is continued.

In an exemplary embodiment, when the computer program is executed by the processor, the step of calculating a maximum current limit for the ablation process according to parameters of electrode leads in the ablation catheter 100 may be performed.

In particular, the maximum current limit may be calculated according to Eqn. (13) below:

$$I = \sqrt{\frac{\Delta T cm}{Rt}} \qquad (13)$$

where $\Delta T$ is a temperature change value (measured in °C), *c* is the specific heat capacity of the electrode leads, and *m* is the mass of the leads (measured in g).

If the electrode lead are made of pure copper, then the specific heat capacity c, the mass *m* and the resistance *R* will be 0.39J/(g·°C), 0.15 g and 3 ohms, respectively. Assuming insulating layers of the electrode leads can withstand a maximum temperature of 250 °C and the duration of ablation is 0.01 s, the maximum current limit can be calculated to be 20 A according to Eqn. (13). Accordingly, if a current used in the ablation process does not exceed 20 A, insulation failure of the electrode leads can be substantially prevented during the ablation process, which may lead to thermal damage to the target tissue. In this way, safety of the ablation process can be effectively improved.

In an exemplary embodiment, when the computer program is executed by the processor, the step of calculating a minimum resistance limit for the ablation process according to the maximum current limit may be carried out.

An initial temperature of the target tissue may be 37 °C, and the specific heat capacity and density of the human body are close to those of water, which are 4.2 J/(g·°C) and 1 g/cm$^3$, respectively. Assuming the target tissue can withstand a maximum temperature of 60 °C, the duration of ablation is 0.004 s, a volume of the target tissue to be ablated is 1 cm$^3$ and the maximum current limit is 20 A, the minimum resistance limit for the ablation process can be calculated to be 60 ohms according to Eqn. (13).

In an exemplary embodiment, when the computer program is executed by the processor, the step of determining whether the resistance exceeds the minimum resistance limit in real time during the ablation process may be performed.

Specifically, as can be seen from Eqn. (13), in the ablation process, when the resistance drops below the minimum resistance limit (e.g., 60 ohms), the ablation current will exceed the maximum current limit (e.g., 20A). This may lead to insulation failure of the electrode leads, which may in turn lead to thermal damage to the target tissue. Accordingly, through determining in real time whether the resistance exceeds the minimum resistance limit during the ablation process, insulation failure of the electrode leads and hence thermal damage to the target tissue can be effectively avoided. Notably, as would be appreciated by those skilled in the art, whenever the resistance drops below the minimum resistance limit, the ablation must be creased immediately.

Some specific exemplary embodiments will be set forth below to further explain the benefits of the readable storage medium provided in the present invention.

## Embodiment 1

In embodiment 1, a target ablation lesion depth was 4 mm, and the first predetermined range was set to be -0.5 mm to 0.5 mm. Through a parameter configuration module in an apparatus, the operator carried out parameter configuration as follows: two-phase rectangular-wave pulses; peak pulse voltage = 2000 V; pulse width = 5 μs; duration of ablation = 0.010 s. As the pulse dose *Dose* was calculated by a data processing module in the apparatus to be within the second predetermined range (e.g., 800-2000 $V^2 \cdot s$), the resistance was then detected and compared with the second threshold (e.g., 60 ohms) to check if it exceeded the second threshold. The resistance was detected to be 167 ohms, greater than 60 ohms. Accordingly, the contact force was then detected and compared with the first threshold (e.g., 5 gf) to check if it was greater than or equal to the first threshold. The contact force was detected to be equal to 5 gf, and therefore ablation was commenced with electric field energy of 36 J. In embodiment 1, the effective energy utilization rate C was not introduced to the calculation of theoretical ablation lesion volume. That is, theoretical ablation lesion volume was calculated according to $V(T) = K \cdot \int_0^T R_t^{\alpha} \cdot W_t^{\beta} \cdot F_t^{\gamma} dt$, and theoretical ablation lesion depth was calculated according to Eqn. (11). The change value of resistance over the process was 16 ohms, and theoretical ablation lesion depth was calculated to be 4.5 mm. Since the difference between theoretical and target ablation lesion depths was within the first predetermined range, the ablation process was stopped. The resulting ablation lesion was then actually measured to have an ablation lesion depth of 3.5 mm.

## Embodiment 2

In embodiment 2, a target ablation lesion depth was 4 mm, and the first predetermined range was set to be -0.5 mm to 0.5 mm. Through a parameter configuration module in an apparatus, the operator carried out parameter configuration as follows: two-phase rectangular-wave pulses; peak pulse voltage = 2000 V; pulse width = 5 μs; duration of ablation = 0.010 s. As the pulse dose *Dose* was calculated by a data processing module in the apparatus to be within the second predetermined range (e.g., 800 $V^2 \cdot s$), the resistance was then detected and compared with the second threshold (e.g., 60 ohms) to check if it exceeded the second threshold. The resistance was detected to be 164 ohms, greater than 60 ohms. Accordingly, the contact force was then detected and compared with the first threshold (e.g., 5 gf) to check if it was greater than or equal to the first threshold. The contact force was detected to be equal to 7 gf, greater than 5 gf, and therefore ablation was commenced with electric field energy of 67 J. During the ablation process, the effective energy utilization rate C was calculated according to the change value of resistance, and theoretical ablation lesion volume was calculated according to Eqns. (1) and (11). The change value of resistance over the process was 21 ohms, and theoretical ablation lesion depth was calculated to be 4.3 mm. Since the difference between theoretical and target ablation lesion depths was within the first predetermined range, the ablation process was stopped. The resulting ablation lesion was then actually measured to have an ablation lesion depth of 4.1 mm. Thus, through using the equation provided herein to calculate theoretical ablation lesion size, it can be ensured that the actual size of the resulting ablation lesion is close to theoretical ablation lesion size and hence to the target ablation lesion size.

**Embodiment 3**

In embodiment 3, a target ablation lesion depth was 5 mm, and the first predetermined range was set to be -0.5 mm to 0.5 mm. Through a parameter configuration module in an apparatus, the operator carried out parameter configuration as follows: two-phase rectangular-wave pulses; peak pulse voltage = 1500 V; pulse width = 2 $\mu$s; duration of ablation = 0.020 s. As the pulse dose *Dose* was calculated by a data processing module in the apparatus to be within the second predetermined range (e.g., 800-2000 $V^2 \cdot s$), the resistance was then detected and compared with the second threshold (e.g., 60 ohms) to see if it exceeded the second threshold. The resistance was detected to be 175 ohms, greater than 60 ohms. Accordingly, the contact force was then detected and compared with the first threshold (e.g., 5 gf) to see if it was greater than or equal to the first threshold. The contact force was detected to be equal to 9 gf, greater than 5 gf, and therefore ablation was commenced with electric field energy of 120 J. During the ablation process, theoretical ablation lesion depth is calculated in real time according to the change value of resistance and Eqns. (1) and (11). The change value of resistance over the process was 30 ohms, and theoretical ablation lesion depth was calculated to be 5.5 mm. Since the difference between theoretical and target ablation lesion depths was within the first predetermined range, the ablation process was stopped. The resulting ablation lesion was then actually measured to have an ablation lesion depth of 5.3 mm. In embodiment 3, the effective energy utilization rate C was first calculated according to the change value of resistance, and theoretical ablation lesion size was then in turn calculated according to the effective energy utilization rate C. Therefore, the actual size of the resulting ablation lesion was close to theoretical ablation lesion size and hence to the target ablation lesion size.

As can be seen from Embodiments above, although it is feasible to predict and control the ablation lesion depth according to the method provided in the present disclosure without introducing the effective energy utilization rate C, the prediction accuracy is suboptimal and the difference between the actual and theoretical ablation lesion depths is relatively large. Moreover, when through introducing the effective energy utilization rate C to the calculation equations, the difference between the actual and theoretical ablation lesion depths becomes smaller, making the prediction more accurate.

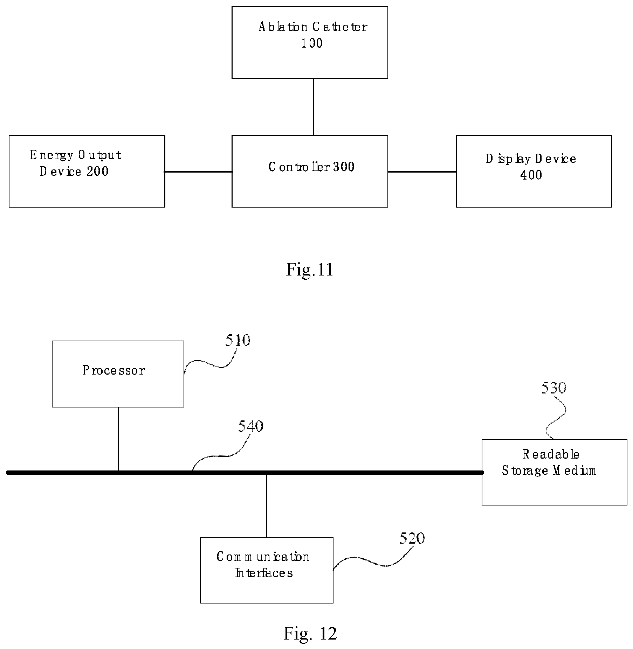

The present disclosure further provides an ablation system corresponding to the above-discussed readable storage medium. Fig. 11 is a schematic block diagram illustrating the structure of an ablation system according to an embodiment of the present disclosure.

As shown in Fig. 11, the ablation system includes an ablation catheter 100, an energy output device 200 and a controller 300. Each of the ablation catheter 100 and the energy output device 200 is communicatively connected to the controller 300. The energy output device 200 is configured to output pulsed energy to the ablation catheter 100. The controller 300 comprises the readable storage medium discussed above. Therefore, during ablation, the ablation system of the present disclosure can calculate a theoretical ablation lesion size in real time and compare it with a target ablation lesion size, thus allowing prediction of ablation efficacy. This can effectively enhance the success rate of the ablation procedure and avoid irreversible thermal damage from being caused to tissue.

In particular, the energy output device 200 may be a pulse generator configured to provide the ablation catheter 100 with a pulsed current for generating a monopolar or bipolar pulsed discharge, in order to achieve ablation.

In an exemplary embodiment, the ablation catheter 100 may be provided at a distal end thereof with a pressure sensor for detecting the magnitude and direction of a contact force between the distal end of the ablation catheter 100 and target tissue. The controller may also be configured to derive an angle of a fit between the distal end of the ablation catheter and the target tissue based on the direction of the contact force between the distal end of the ablation catheter and the target tissue detected by the pressure sensor. With this arrangement, in a practical ablation process, precision ablation can be achieved by controlling snugness of the fit between the distal end of the ablation catheter 100 and the target tissue (which depends on the magnitude of the contact force) and the angle of the fit (which depends on the direction of the contact force).

In an exemplary embodiment, the ablation catheter 100 may be provided at the distal end thereof with a temperature sensor for detecting a temperature of the target tissue being ablated. Irreversible thermal damage may be caused to the target tissue at an excessively high temperature. Therefore, the temperature sensor may be provided to detect the temperature of the target tissue in real time during ablation, thereby ensuring that no irreversible thermal damage is caused to the target tissue. Notably, as would be appreciated by those skilled in the art, when detecting that the ablation temperature of the target tissue is excessively high, the tissue may be cooled by perfusion with saline at a low flow rate, so as to enable ablation temperature of the tissue to return to an appropriate temperature range.

With continued reference to Fig. 11, in an exemplary embodiment, the ablation system may further include a display device 400 communicatively connected to the controller 300, which can display in real time the resistance, the magnitude and direction of the contact force between the distal end of the ablation catheter 100 and the target tissue, the angle of the fit therebetween and the ablation temperature of target tissue, helping the operator optimize the fit and achieve precision ablation. In particular, the angle of the fit between the distal end of the ablation catheter 100 and the target tissue may be: an angle between an axis of the distal end of the ablation catheter 100 and the direction of the contact force minus 90°.

Further, a 3D model of the ablation catheter and target tissue may be built, so that, as shown in Figs. 4 and 5, the magnitude of the contact force between the distal end of the ablation catheter 100 and the target tissue (i.e., the snugness fit established therebetween), and the direction of the contact force and the angle of the fit may be displayed on the 3D model of the ablation catheter and target tissue.

On the basis of the same inventive concept, the present disclosure also provides an electronic device. Fig. 12 is a schematic block diagram illustrating the structure of the electronic device according to an embodiment of the present invention. As shown in Fig. 12, the electronic device includes a processor 510 and the readable storage medium 530 as defined above. Since the electronic device is based on the same inventive concept as the above-discussed readable storage medium, it has all the advantages of the readable storage medium. Reference can be made to the above description for more details of the benefits of the electronic device, and further description thereof is omitted here for the sake of brevity.

As shown in Fig. 12, the electronic device may further include communication interfaces 520 and communication buses 540. Communications between the processor 510, the communication interfaces 520 and the readable storage medium 530 may be accomplished through the communication buses 540. Each of the communication bus 540 may be, for example, a peripheral component interconnect (PCI) bus or an extended industry standard architecture (EISA) bus. The communication buses 540 may include address buses, data buses, control buses, etc. Although they are represented by only one bold line in the figure for ease of illustration, this does not mean that there is only one bus, or only one type of bus. The communication interfaces 520 are configured to enable communications of the electronic device with other devices.

The processor 510 suitable for use in this invention may be a central processing unit (CPU) or other general-purpose processor, a digital signal processor (DSP), an application specific integrated circuit (ASIC), a field-programmable gate array (FPGA) or other programmable logic device, a discrete gate or transistor-transistor logic device, a discrete hardware component, or the like. The general-purpose processor may be a microprocessor or any other conventional processor. The processor 510 may serve as a control center of the electronic device, and the various components in the electronic device may be interconnected with various interfaces and lines.

The processor 510 can perform various functions of the electronic device by running or executing the computer program stored in the readable storage medium 530 and retrieving data stored in the readable storage medium 530.

Notably, as would be appreciated by those skilled in the art, computer program code for carrying out operations of the present invention may be written in any combination of one or more programming languages, including an object-oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on a remote computer or server. In relation to the remote computer, the remote computer may be connected to the user's computer through any type of network, including a local area network (LAN) or a wide area network (WAN), or the connection may be made to an external computer (for example, through the Internet using an Internet Service Provider).

In summary, the readable storage medium, the ablation system and the electronic device provided in the present disclosure have the following advantages over the prior art: when the computer program stored in the readable storage medium is executed by the processor, the following steps are carried out: controlling the ablation catheter to apply pulsed energy to target tissue and obtaining electric field energy and resistance in the ablation process; calculating a theoretical ablation lesion size according to the electric field energy and the resistance and determining whether the difference between theoretical ablation lesion size and a target ablation lesion size is within a first predetermined range; if so, stopping applying pulsed energy to the target tissue; if not, continuing applying pulsed energy to the target tissue, until the difference between theoretical and target ablation lesion sizes is within the first predetermined range. In this way, according to the present invention, the theoretical ablation lesion size is calculated and compared with the target ablation lesion size in real time during the PFA process, thus allowing prediction of PFA efficacy. This can effectively enhance the success rate of the PFA procedure and avoid irreversible thermal damage from being caused to the tissue.

Since the ablation system and electronic device provided in the present disclosure is based on the same inventive concept as the readable storage medium provided in the present disclosure, they have all the advantages of the readable storage medium. Reference can be made to the above description for more details of the benefits of the ablation system and the electronic device, and further description thereof is omitted here for the sake of brevity.

It is noted that the devices and methods disclosed in the embodiments herein may also be implemented in other ways and the device embodiments described above are merely illustrative. For example, the flowcharts and block diagrams in the figures illustrate the architecture, functionality and operation of possible implementations of devices, methods, and computer program products according to various embodiments of the present disclosure.

In this regard, each block in the flowcharts or block diagrams may represent a module, program, or portion of instructions, which comprises one or more executable instructions for implementing the specified logical function(s). It is also noted that, in some alternative implementations, the functions noted in the blocks may occur out of the order noted in the figures. For example, two blocks shown in succession may, in fact, be executed substantially concurrently, or the blocks may sometimes be executed in the reverse order, depending upon the functionality involved. It is further noted that each block of the block diagrams and/or flowchart illustrations, and combinations of blocks in the block diagrams and/or flowchart illustrations, can be implemented by special purpose hardware-based systems that perform the specified functions or actions or may be carried out by combinations of special purpose hardware and computer instructions.

Further, the various functional modules in the embodiments herein may be integrated into a discrete component, or provided as separate modules. Alternatively, two or more of the modules may be integrated into a discrete component.

The description presented above is merely that of some preferred embodiments of the present invention and is not intended to limit the scope thereof. Any and all changes and modifications made by those of ordinary skill in the art based on the above teachings fall within the scope as defined in the appended claims. Apparently, those skilled in the art can make various modifications and variations to the present invention without departing from the scope thereof. Accordingly, the invention is intended to comprise all such modifications and variations if they fall within the scope of the appended claims and equivalents thereof.

## Claims

1.  A readable storage medium (530) having a computer program stored thereon, wherein the computer program is executed by a processor (510) to perform the steps of:

    controlling an ablation catheter (100) to apply pulsed energy to a target tissue and determining electric field energy and a resistance during an ablation process (S100);
    calculating a theoretical ablation lesion size (S200);
    determining whether a difference between the theoretical ablation lesion size and a target ablation lesion size is within a first predetermined range (S300);
    if so, controlling the ablation catheter (100) to stop applying the pulsed energy to the target tissue (S400); and
    if not, controlling the ablation catheter (100) to continue applying the pulsed energy to the target tissue, until the difference between the theoretical and target ablation lesion sizes is within the first predetermined range, **characterized in that**,
    when executed by the processor (510), the computer program further performs the step of:

    determining an effective energy utilization rate during the ablation process,
    wherein calculating the theoretical ablation lesion size (S200) comprises:
    calculating the theoretical ablation lesion size according to the electric field energy, the resistance and the effective energy utilization rate.

2.  The readable storage medium (530) according to claim 1, i) wherein determining the effective energy utilization rate during the ablation process comprises:

    determining a change value of the resistance during the ablation process; and
    determining the effective energy utilization rate according to the change value of the resistance and the following equation:

    $$C = \frac{R_0 - R_T}{R_0}$$

    where $C$ is the effective energy utilization rate, $R_0$ is a resistance before start of the ablation process, $R_T$ is a resistance at time $T$ after start of the ablation process, and $(R_0 - R_T)$ is the change value of resistance; or
    ii) wherein the effective energy utilization rate is a predetermined value in a range of 0. 15-0.35.

3.  The readable storage medium (530) according to claim 1, wherein a pressure sensor is provided at a distal end of the ablation catheter (100), wherein the pressure sensor is configured to detect a magnitude and a direction of a contact force between the distal end of the ablation catheter (100) and the target tissue,
    wherein determining the effective energy utilization rate of the ablation process comprises:

    determining an angle of a fit established between the distal end of the ablation catheter (100) and the target tissue according to the direction of the contact force between the distal end of the ablation catheter (100) and the target tissue detected by the pressure sensor; and

determining the effective energy utilization rate according to the angle of the fit between the distal end of the ablation catheter (100) and the target tissue, wherein the effective energy utilization rate increases with a decrease in the angle of the fit between the distal end of the ablation catheter (100) and the target tissue.

4. The readable storage medium (530) according to claim 1, wherein calculating the theoretical ablation lesion size according to the electric field energy, the resistance and the effective energy utilization rate comprises:
calculating the theoretical ablation lesion size according to the electric field energy, the resistance, the effective energy utilization rate and a magnitude of a contact force between the distal end of the ablation catheter (100) and the target tissue.

5. The readable storage medium (530) according to claim 4, wherein calculating the theoretical ablation lesion size according to the electric field energy, the resistance, the effective energy utilization rate and the magnitude of the contact force between the distal end of the ablation catheter and the target tissue comprises:

calculating a theoretical ablation lesion volume according to the following equation:

$$V(T) = K \cdot C \cdot \int_0^T R_t^{\alpha} \cdot W_t^{\beta} \cdot F_t^{\gamma} dt$$

where $V(T)$ is the theoretical ablation lesion volume at time $T$ after start of the ablation process, $C$ is the effective energy utilization rate, $R_t$ is the resistance at time $T$ after start of the ablation process, $W_t$ is an electric field energy at time $T$ after start of the ablation process, $F_t$ is the magnitude of the contact force between the distal end of the ablation catheter and the target tissue at time $T$ after start of the ablation process, $K$ is a scaling constant, and each of $\alpha$, $\beta$ and $\gamma$ is an exponent not equal to 1.

6. The readable storage medium (530) according to claim 5, wherein the theoretical ablation lesion size is the theoretical ablation lesion volume, and the target ablation lesion size is a target ablation lesion volume.

7. The readable storage medium (530) according to claim 5, wherein when executed by the processor (510), the computer program further performs the step of:

calculating a theoretical ablation lesion depth according to the theoretical ablation lesion volume and the following equation:

$$V = kD^3$$

where $D$ is the theoretical ablation lesion depth, $k$ is a scaling constant, wherein the theoretical ablation lesion size is the theoretical ablation lesion depth, and the target ablation lesion size is a target ablation lesion depth.

8. The readable storage medium (530) according to claim 1, wherein when executed by the processor (510), the computer program further performs the step of:

before the ablation catheter is controlled to apply the pulsed energy to the target tissue, determining whether a fit is established between the distal end of the ablation catheter (100) and the target tissue, optionally wherein a pressure sensor is provided at a distal end of the ablation catheter (100), wherein the pressure sensor is configured to detect a contact force between the distal end of the ablation catheter (100) and the target tissue, wherein determining whether the fit is established between the distal end of the ablation catheter (100) and the target tissue comprises:

determining whether a magnitude of the contact force detected by the pressure sensor is greater than or equal to a first threshold; and
if so, determining that the fit has been established between the distal end of the ablation catheter and the target tissue.

9. The readable storage medium (530) according to claim 1, wherein when executed by the processor (510), the computer program further performs the step of:
before the ablation catheter (100) is controlled to apply the pulsed energy to the target tissue, determining whether a short is present in a circuit between an energy output device (200) for supplying the pulsed energy to the ablation catheter (100) and the ablation catheter (100), optionally wherein determining whether the short is present in the circuit between the energy output device (200) for supplying the pulsed energy to the ablation catheter (100) and the ablation catheter (100) comprises:

determining whether the resistance before start of the ablation process exceeds a second threshold; and
if not, determining that the short is present in the circuit between the energy output device (200) and the ablation catheter (100).

10. The readable storage medium (530) according to claim 1, wherein when executed by the processor (510), the computer program further performs the steps of:

before the ablation catheter (100) is controlled to the apply pulsed energy to the target tissue, configuring pulse parameters and calculating a pulse dose according to the pulse parameters;
determining whether the pulse dose is within a second predetermined range; and
if not, reconfiguring the pulse parameters until the pulse dose is within the second predetermined range, optionally wherein calculating the pulse dose according to the pulse parameters comprises:
calculating the pulse dose according to the following equation:

$$Dose = U^2 \cdot a \cdot d \cdot b \cdot n$$

where *Dose* is the pulse dose, *U* is a pulse voltage, *a* is a number of pulses in each pulse train, *d* is a number of pulse trains delivered in each ablation cycle, *b* is a pulse width, and *n* is a number of ablation cycles.

11. The readable storage medium (530) according to claim 1, wherein when executed by the processor (510), the computer program further performs the step of:

calculating a maximum current limit for the ablation process according to parameter information of electrode leads in the ablation catheter (100),
optionally wherein when executed by the processor (510), the computer program further performs the step of:

calculating a minimum resistance limit for the ablation process according to the maximum current limit,
further optionally wherein when executed by the processor (510), the computer program further performs the step of:
determining whether the resistance exceeds the minimum resistance limit in real time during the ablation process.

12. An ablation system, comprising an ablation catheter (100), an energy output device (200) and a controller (300), wherein each of the ablation catheter (100) and the energy output device (200) is communicatively connected to the controller (300), wherein the energy output device (200) is configured to output pulsed energy to the ablation catheter (100), and wherein the controller (300) comprises the readable storage medium (530) according to any one of claims 1 to 11.

13. The ablation system according to claim 12, i) wherein a pressure sensor is provided at a distal end of the ablation catheter (100), wherein the pressure sensor is configured to detect a magnitude and a direction of a contact force between the distal end of the ablation catheter (100) and a target tissue,

wherein the controller (300) is further configured to: determine an angle of a fit established between the distal end of the ablation catheter (100) and the target tissue according to the direction of the contact force between the distal end of the ablation catheter (100) and the target tissue detected by the pressure sensor; and
wherein the ablation system further comprises a display device (400) communicatively connected to the controller (300), wherein the display device (400) is configured to display in real time a resistance, the magnitude and direction of the contact force between the distal end of the ablation catheter (100) and the target tissue, and an angle of the fit between the distal end of the ablation catheter (100) and the target tissue; or
ii) wherein a temperature sensor is further provided at the distal end of the ablation catheter (100), wherein the temperature sensor is configured to detect an ablation temperature of the target tissue.

14. An electronic device, comprising a processor (510) and the readable storage medium (530) according to any one of claims 1 to 13.

**Patentansprüche**

1. Ein lesbares Speichermedium (530), auf dem ein Computerprogramm gespeichert ist, wobei das Computerprogramm von einem Prozessor (510) ausgeführt wird, um folgende Schritte durchzuführen:

   Steuerung eines Ablationskatheters (100) zur Anwendung gepulster Energie auf ein Zielgewebe und Bestimmung elektrischer Feldenergie und eines Widerstands während eines Ablationsprozesses (S100);
   Berechnung einer theoretischen Ablationsläsionsgröße (S200);
   Feststellung, ob eine Differenz zwischen der theoretischen Ablationsläsionsgröße und der Zielablationsläsionsgröße innerhalb eines ersten vorgegebenen Bereichs liegt (S300);
   Falls ja, Steuerung des Ablationskatheters (100), um die Anwendung der gepulsten Energie auf das Zielgewebe zu beenden (S400); und
   Falls nicht, Steuerung des Ablationskatheters (100), um die Anwendung der gepulsten Energie auf das Zielgewebe fortzusetzen, bis die Differenz zwischen der theoretischen Ablationsläsionsgröße und der Zielablationsläsionsgröße innerhalb des ersten vorbestimmten Bereichs liegt, **dadurch gekennzeichnet, dass**
   bei der Ausführung durch den Prozessor (510), das Computerprogramm ferner den Schritt ausführt:

   Ermittlung eines effektiven Energieausnutzungsgrades während des Ablationsprozesses,
   wobei die Berechnung der theoretischen Ablationsläsionsgröße (S200) umfasst:
   Berechnung der theoretischen Ablationsläsionsgröße anhand der elektrischen Feldenergie, des Widerstands und des effektiven Energieausnutzungsgrades.

2. Das lesbare Speichermedium (530) nach Anspruch 1, i) wobei die Bestimmung des effektiven Energieausnutzungsgrades während des Ablationsprozesses Folgendes umfasst:

   Bestimmung eines Änderungswerts des Widerstands während des Ablationsprozesses; und
   Bestimmung des effektiven Energieausnutzungsgrades gemäß dem Änderungswert des Widerstands und der folgenden Gleichung:

   $$C = \frac{R_0 - R_T}{R_0}$$

   wobei C der effektive Energieausnutzungsgrad ist, $R_0$ ein Widerstand vor Beginn des Ablationsprozesses ist, $R_T$ ein Widerstand zum Zeitpunkt $T$ nach Beginn des Ablationsprozesses ist, und ($R_0$ - $R_T$) der Änderungswert des Widerstands ist; oder
   ii) wobei der effektive Energieausnutzungsgrad ein vorbestimmter Wert in einem Bereich von 0,15 bis 0,35 ist.

3. Das lesbare Speichermedium (530) nach Anspruch 1, wobei an einem distalen Ende des Ablationskatheters (100) ein Drucksensor vorgesehen ist, wobei der Drucksensor so konfiguriert ist, dass er eine Größe und eine Richtung einer Kontaktkraft zwischen dem distalen Ende des Ablationskatheters (100) und dem Zielgewebe erfasst,
   wobei die Bestimmung des effektiven Energieausnutzungsgrades des Ablationsprozesses Folgendes umfasst:

   Bestimmung eines Winkels einer Anpassung, die zwischen dem distalen Ende des Ablationskatheters (100) und dem Zielgewebe gemäß der Richtung der durch den Drucksensor erfassten Kontaktkraft zwischen dem distalen Ende des Ablationskatheters (100) und dem Zielgewebe hergestellt ist; und
   Bestimmung des effektiven Energieausnutzungsgrades gemäß dem Winkel der Anpassung zwischen dem distalen Ende des Ablationskatheters (100) und dem Zielgewebe, wobei der effektive Energieausnutzungsgrad mit einer Verringerung des Winkels der Anpassung zwischen dem distalen Ende des Ablationskatheters (100) und dem Zielgewebe zunimmt.

4. Das lesbare Speichermedium (530) nach Anspruch 1, wobei die Berechnung der theoretischen Ablationsläsionsgröße gemäß der elektrischen Feldenergie, dem Widerstand und dem effektiven Energieausnutzungsgrad Folgendes umfasst:
Berechnung der theoretischen Ablationsläsionsgröße gemäß der elektrischen Feldenergie, dem Widerstand, dem effektiven Energieausnutzungsgrad und einer Größe einer Kontaktkraft zwischen dem distalen Ende des Ablationskatheters (100) und dem Zielgewebe.

5. Das lesbare Speichermedium (530) nach Anspruch 4, wobei die Berechnung der theoretischen Ablationsläsionsgröße gemäß der elektrischen Feldenergie, dem Widerstand, dem effektiven Energieausnutzungsgrad und der Größe der Kontaktkraft zwischen dem distalen Ende des Ablationskatheters und dem Zielgewebe Folgendes umfasst:

Berechnung eines theoretischen Ablationsläsionsvolumens gemäß der folgenden Gleichung:

$$V(T) = K \cdot C \cdot \int_0^T R_t^{\alpha} \cdot W_t^{\beta} \cdot F_t^{\gamma} \, dt$$

wobei $V(T)$ das theoretische Ablationsläsionsvolumen zum Zeitpunkt $T$ nach Beginn des Ablationsprozesses ist, $C$ der effektive Energieausnutzungsgrad ist, $R$, der Widerstand zum Zeitpunkt $T$ nach Beginn des Ablationsprozesses ist, $W_t$ die elektrische Feldenergie zum Zeitpunkt $T$ nach Beginn des Ablationsprozesses ist, $F$, die Größe der Kontaktkraft zwischen dem distalen Ende des Ablationskatheters und dem Zielgewebe zum Zeitpunkt $T$ nach Beginn des Ablationsprozesses ist, $K$ eine Skalierungskonstante ist, und jeder von $\alpha$, $\beta$ und $\gamma$ ein Exponent ist, der nicht gleich 1 ist.

6. Das lesbare Speichermedium (530) nach Anspruch 5, wobei die theoretische Ablationsläsionsgröße das theoretische Ablationsläsionsvolumen ist und die Zielablationsläsionsgröße ein Zielablationsläsionsvolumen ist.

7. Das lesbare Speichermedium (530) nach Anspruch 5, wobei, wenn es durch den Prozessor (510) ausgeführt wird, das Computerprogramm ferner den Schritt ausführt:

Berechnung einer theoretischen Ablationsläsionstiefe gemäß dem theoretischen Ablationsläsionsvolumen und der folgenden Gleichung:

$$V = kD^3$$

wobei $D$ die theoretische Ablationsläsionstiefe ist, $k$ eine Skalierungskonstante ist, wobei die theoretische Ablationsläsionsgröße die theoretische Ablationsläsionstiefe ist, und die Zielablationsläsionsgröße eine Zielablationläsionstiefe ist.

8. Das lesbare Speichermedium (530) nach Anspruch 1, wobei, wenn es durch den Prozessor (510) ausgeführt wird, das Computerprogramm ferner den Schritt ausführt:

vor der Steuerung des Ablationskatheters (100) zur Anwendung der gepulsten Energie auf das Zielgewebe, Feststellung, ob eine Anpassung zwischen dem distalen Ende des Ablationskatheters (100) und dem Zielgewebe hergestellt ist, optional wobei an einem distalen Ende des Ablationskatheters (100) ein Drucksensor vorgesehen ist, wobei der Drucksensor so konfiguriert ist, dass er eine Kontaktkraft zwischen dem distalen Ende des Ablationskatheters (100) und dem Zielgewebe erfasst,
wobei die Feststellung, ob die Anpassung zwischen dem distalen Ende des Ablationskatheters (100) und dem Zielgewebe hergestellt ist, Folgendes umfasst:

Bestimmung, ob eine von dem Drucksensor erfasste Größe der Kontaktkraft größer als oder gleich einem ersten Schwellenwert ist; und
falls ja, Feststellung, dass die Anpassung zwischen dem distalen Ende des Ablationskatheters (100) und dem Zielgewebe hergestellt wurde.

9. Das lesbare Speichermedium (530) nach Anspruch 1, wobei, wenn es durch den Prozessor (510) ausgeführt wird, das Computerprogramm ferner den Schritt ausführt:
vor der Steuerung des Ablationskatheters (100) zur Anwendung der gepulsten Energie auf das Zielgewebe, Feststellung, ob ein Kurzschluss in einem Stromkreis zwischen einer Energieabgabevorrichtung (200) zum Zuführen der gepulsten Energie an den Ablationskatheter (100) und dem Ablationskatheter (100) vorliegt, optional wobei die Feststellung, ob der Kurzschluss in dem Stromkreis zwischen der Energieabgabevorrichtung (200) und dem Ablationskatheter (100) vorliegt, Folgendes umfasst:

Bestimmung, ob der Widerstand vor Beginn des Ablationsprozesses einen zweiten Schwellenwert überschreitet; und

falls nicht, Feststellung, dass der Kurzschluss in dem Stromkreis zwischen der Energieabgabevorrichtung (200) und dem Ablationskatheter (100) vorliegt.

10. Das lesbare Speichermedium (530) nach Anspruch 1, wobei, wenn es durch den Prozessor (510) ausgeführt wird, das Computerprogramm ferner die folgenden Schritte ausführt:

vor der Steuerung des Ablationskatheters (100) zur Anwendung der gepulsten Energie auf das Zielgewebe, Konfiguration von Pulsparametern und Berechnung einer Pulsdosis gemäß den Pulsparametern;
Bestimmung, ob die Pulsdosis innerhalb eines zweiten vorbestimmten Bereichs liegt; und
falls nicht, Neukonfiguration der Pulsparameter, bis die Pulsdosis innerhalb des zweiten vorbestimmten Bereichs liegt,
optional wobei die Berechnung der Pulsdosis gemäß den Pulsparametern Folgendes umfasst:
Berechnung der Pulsdosis gemäß der folgenden Gleichung:

$$Dose = U^2 \cdot a \cdot d \cdot b \cdot n$$

Wobei *Dose* die Pulsdosis ist, *U* eine Pulsspannung ist, *a* die Anzahl der Pulse in jeder Pulsfolge ist, *d* die Anzahl der Pulsfolgen ist, die in jedem Ablationszyklus abgegeben werden, *b* eine Pulsbreite ist, und *n* die Anzahl der Ablationszyklen ist.

11. Das lesbare Speichermedium (530) nach Anspruch 1, wobei, wenn es durch den Prozessor (510) ausgeführt wird, das Computerprogramm ferner den Schritt ausführt:

Berechnung einer maximalen Stromgrenze für den Ablationsprozess gemäß Parameterinformationen von Elektrodenleitungen in dem Ablationskatheter (100),
optional wobei, wenn es durch den Prozessor (510) ausgeführt wird, das Computerprogramm ferner den Schritt ausführt:

Berechnung einer minimalen Widerstandsgrenze für den Ablationsprozess gemäß der maximalen Stromgrenze,
ferner optional wobei, wenn es durch den Prozessor (510) ausgeführt wird, das Computerprogramm ferner den Schritt ausführt:
Bestimmung, ob der Widerstand während des Ablationsprozesses in Echtzeit die minimale Widerstandsgrenze überschreitet.

12. Ein Ablationssystem, umfassend einen Ablationskatheter (100), eine Energieabgabevorrichtung (200) und einen Steuercontroller (300), wobei sowohl der Ablationskatheter (100) als auch die Energieabgabevorrichtung (200) mit dem Steuercontroller (300) kommunikationsmäßig verbunden sind, wobei die Energieabgabevorrichtung (200) so konfiguriert ist, dass sie gepulste Energie an den Ablationskatheter (100) ausgibt, und wobei der Steuercontroller (300) das lesbare Speichermedium (530) nach einem der Ansprüche 1 bis 11 umfasst.

13. Das Ablationssystem nach Anspruch 12, i) wobei an einem distalen Ende des Ablationskatheters (100) ein Drucksensor vorgesehen ist, wobei der Drucksensor so konfiguriert ist, dass er eine Größe und eine Richtung einer Kontaktkraft zwischen dem distalen Ende des Ablationskatheters (100) und einem Zielgewebe erfasst,

wobei der Steuercontroller (300) ferner konfiguriert ist, einen Winkel einer Anpassung, die zwischen dem distalen Ende des Ablationskatheters (100) und dem Zielgewebe gemäß der Richtung der durch den Drucksensor erfassten Kontaktkraft zwischen dem distalen Ende des Ablationskatheters (100) und dem Zielgewebe hergestellt ist, zu bestimmen; und
wobei das Ablationssystem ferner eine Anzeigevorrichtung (400) umfasst, die kommunikationsmäßig mit dem Steuercontroller (300) verbunden ist, wobei die Anzeigevorrichtung (400) konfiguriert ist, in Echtzeit einen Widerstand, die Größe und die Richtung der Kontaktkraft zwischen dem distalen Ende des Ablationskatheters (100) und dem Zielgewebe, sowie einen Winkel der Anpassung zwischen dem distalen Ende des Ablationskatheters (100) und dem Zielgewebe anzuzeigen; oder

ii) wobei an dem distalen Ende des Ablationskatheters (100) ferner ein Temperatursensor vorgesehen ist, wobei der Temperatursensor konfiguriert ist, eine Ablationstemperatur des Zielgewebes zu erfassen.

**14.** Eine elektronische Vorrichtung, umfassend einen Prozessor (510) und das lesbare Speichermedium (530) nach einem der Ansprüche 1 bis 13.

## Revendications

**1.** Un support de stockage lisible (530) sur lequel est stocké un programme informatique, dans lequel le programme informatique est exécuté par un processus ou (510) pour effectuer les étapes suivantes :

contrôler un cathéter d'ablation (100) pour appliquer une énergie pulsée à un tissu cible et déterminer l'énergie du champ électrique et une résistance pendant un processus d'ablation (S100) ;
calcul de la taille théorique d'une lésion d'ablation (S200) ;
déterminer si une différence entre la taille théorique de la lésion d'ablation et la taille cible de la lésion d'ablation se situe dans une première plage prédéterminée (S300) ;
si oui, contrôler le cathéter d'ablation (100) pour arrêter d'appliquer l' énergie pulsée au tissu cible (S400) ; et
sinon, contrôler le cathéter d'ablation (100) pour continuer à appliquer l' énergie pulsée au tissu cible, jusqu'à ce que la différence entre les tailles théorique et cible de la lésion d'ablation soit dans la première plage prédéterminée, **caractérisée en ce que**,
Lorsqu'il est exécuté par le processeur (510), le programme informatique effectue ensuite l'étape suivante :

déterminer un taux d'utilisation efficace de l'énergie pendant le processus d'ablation,
dans lequel le calcul de la taille théorique de la lésion d'ablation (S200) comprend :
calcul de la taille théorique de la lésion d'ablation en fonction de l'énergie du champ électrique, de la résistance et du taux d'utilisation effectif de l'énergie.

**2.** Le support de stockage lisible (530) selon la revendication 1, i) dans lequel la détermination du taux d'utilisation d'énergie effectif pendant le processus d'ablation comprend :

déterminer la variation de la résistance au cours du processus d'ablation ; et
détermination du taux d'utilisation effective de l'énergie en fonction de la variation de la résistance et de l'équation suivante :

$$C = \frac{R_0 - R_T}{R_0}$$

où $C$ représente le taux d'utilisation d'énergie effectif, $R_0$ la résistance avant le début du processus d'ablation, $R_T$ la résistance au temps $T$ après le début du processus d'ablation, et ($R_0 - R_T$) la variation de résistance ; ou
ii) dans lequel le taux d'utilisation effective de l'énergie est une valeur prédéterminée dans une plage de 0,15 à 0,35.

**3.** Le support de stockage lisible (530) selon la revendication 1, dans lequel un capteur de pression est prévu à l'extrémité distale du cathéter d'ablation (100), ce capteur de pression étant configuré pour détecter l'amplitude et la direction d'une force de contact entre l'extrémité distale du cathéter d'ablation (100) et le tissu cible,
dans lequel la détermination du taux d'utilisation effective de l'énergie du processus d'ablation comprend :

déterminer un angle d'ajustement établi entre l'extrémité distale du cathéter d'ablation (100) et le tissu cible en fonction de la direction de la force de contact entre l'extrémité distale du cathéter d'ablation (100) et le tissu cible détectée par le capteur de pression ; et
déterminer le taux d'utilisation d'énergie effectif en fonction de l'angle d'ajustement entre l'extrémité distale du cathéter d'ablation (100) et le tissu cible, dans lequel le taux d'utilisation d'énergie effectif augmente avec une diminution de l'angle d'ajustement entre l'extrémité distale du cathéter d'ablation (100) et le tissu cible.

4. Le support de stockage lisible (530) selon la revendication 1, dans lequel le calcul de la taille théorique de la lésion d'ablation en fonction de l'énergie du champ électrique, de la résistance et du taux d'utilisation d'énergie effectif comprend :
calcul de la taille théorique de la lésion d'ablation en fonction de l'énergie du champ électrique, de la résistance, du taux d'utilisation d'énergie effectif et de l' amplitude d'une force de contact entre l'extrémité distale du cathéter d'ablation (100) et le tissu cible.

5. Le support de stockage lisible (530) selon la revendication 4, dans lequel le calcul de la taille théorique de la lésion d'ablation en fonction de l'énergie du champ électrique, de la résistance, du taux d'utilisation d'énergie effectif et de l'amplitude de la force de contact entre l'extrémité distale du cathéter d'ablation et le tissu cible comprend :

calcul du volume théorique d'une lésion d'ablation selon l'équation suivante :

$$V(T) = K \cdot C \cdot \int_0^T R_t^{\alpha} \cdot W_t^{\beta} \cdot F_t^{\gamma} dt$$

où *V(T)* représente le volume théorique de la lésion d'ablation à l'instant *T* après le début du processus d'ablation, *C* le taux d'utilisation effectif de l'énergie, $R_t$ la résistance à l'instant *T* après le début du processus d'ablation, $W_t$ l'énergie du champ électrique à l'instant *T* après le début du processus d'ablation, $F_t$ l'intensité de la force de contact entre l'extrémité distale du cathéter d'ablation et le tissu cible à l'instant *T* après le début du processus d'ablation, *K* une constante d'échelle, et chacun des éléments suivants $\alpha$ , $\beta$ et $\gamma$ est un exposant différent de 1.

6. Le support de stockage lisible (530) selon la revendication 5, dans lequel la taille théorique de la lésion d'ablation est le volume théorique de la lésion d'ablation et la taille cible de la lésion d'ablation est un volume cible de la lésion d'ablation.

7. Le support de stockage lisible (530) selon la revendication 5, dans lequel, lorsqu'il est exécuté par le processeur (510), le programme informatique effectue en outre l'étape suivante :

calcul de la profondeur théorique d'une lésion d'ablation en fonction du volume théorique de la lésion d'ablation et de l'équation suivante :

$$V = kD^3$$

où *D* est la profondeur théorique de la lésion d'ablation, *k* est une constante d'échelle, où la taille théorique de la lésion d'ablation est la profondeur théorique de la lésion d'ablation, et la taille cible de la lésion d'ablation est la profondeur cible de la lésion d'ablation.

8. Le support de stockage lisible (530) selon la revendication 1, dans lequel, lorsqu'il est exécuté par le processeur (510), le programme informatique effectue en outre l'étape suivante :

avant que le cathéter d'ablation ne soit contrôlé pour appliquer l' énergie pulsée au tissu cible, il faut déterminer si un ajustement est établi entre l'extrémité distale du cathéter d'ablation (100) et le tissu cible, éventuellement avec un capteur de pression à l'extrémité distale du cathéter. le cathéter d'ablation (100), dans lequel le capteur de pression est configuré pour détecter une force de contact entre l'extrémité distale du cathéter d'ablation (100) et le tissu cible,
dans lequel la détermination de l' établissement d'un ajustement entre l'extrémité distale du cathéter d'ablation (100) et le tissu cible comprend :

déterminer si l'amplitude de la force de contact détectée par le capteur de pression est supérieure ou égale à un premier seuil ; et
si oui, déterminer que l' ajustement a été établi entre l'extrémité distale du cathéter d'ablation et le tissu cible.

9. Le support de stockage lisible (530) selon la revendication 1, dans lequel, lorsqu'il est exécuté par le processeur (510), le programme informatique effectue en outre l'étape suivante :

avant que le cathéter d'ablation (100) ne soit commandé pour appliquer l' énergie pulsée au tissu cible, déterminer s'il y a un court -circuit dans un circuit entre un dispositif de sortie d'énergie (200) pour fournir l'énergie pulsée au cathéter d'ablation (100) et le cathéter d'ablation (100), éventuellement dans lequel la détermination de la présence d' un court -circuit dans le circuit entre le dispositif de sortie d'énergie (200) destiné à fournir l'énergie pulsée au cathéter d'ablation (100) et le cathéter d'ablation (100) comprend :

déterminer si la résistance avant le début du processus d'ablation dépasse un second seuil ; et
sinon, déterminer que le court -circuit est présent dans le circuit entre le dispositif de sortie d'énergie (200) et le cathéter d'ablation (100).

10. Le support de stockage lisible (530) selon la revendication 1, dans lequel, lorsqu'il est exécuté par le processeur (510), le programme informatique effectue en outre les étapes suivantes :

avant que le cathéter d'ablation (100) ne soit contrôlé pour appliquer une énergie pulsée au tissu cible, en configurant les paramètres d'impulsion et en calculant une dose d'impulsion en fonction des paramètres d'impulsion ;
déterminer si la dose pulsée se situe dans une plage prédéterminée en une seconde ; et
sinon, reconfigurer les paramètres d'impulsion jusqu'à ce que la dose d'impulsion se situe dans la deuxième plage prédéterminée,
optionnellement dans lequel le calcul de la dose pulsée en fonction des paramètres de l'impulsion comprend :

calcul de la dose pulsée selon l'équation suivante :

$$Dose = U^2 \cdot a \cdot d \cdot b \cdot n$$

où *Dose* est la dose d'impulsion, *U* est une tension d'impulsion, *a* est un nombre d'impulsions dans chaque train d'impulsions, *d* est un nombre de trains d'impulsions délivrés dans chaque cycle d'ablation, *b* est une largeur d'impulsion, et *n* est un nombre de cycles d'ablation.

11. Le support de stockage lisible (530) selon la revendication 1, dans lequel, lorsqu'il est exécuté par le processeur (510), le programme informatique effectue en outre l'étape suivante :

calcul d'une limite de courant maximale pour le processus d'ablation en fonction des informations paramétriques des électrodes du cathéter d'ablation (100),
optionnellement dans lequel, lorsqu'il est exécuté par le processeur (510), le programme informatique effectue en outre l'étape suivante :

calcul d'une limite de résistance minimale pour le processus d'ablation en fonction de la limite de courant maximale,
en outre, de manière optionnelle, lorsque le programme informatique est exécuté par le processeur (510), il effectue en outre l'étape suivante :
déterminer en temps réel, pendant le processus d'ablation, si la résistance dépasse la limite de résistance minimale.

12. Un système d'ablation comprenant un cathéter d'ablation (100), un dispositif de sortie d'énergie (200) et un contrôleur (300), chacun du cathéter d'ablation (100) et du dispositif de sortie d'énergie (200) est connecté de manière communicative au contrôleur (300), où le dispositif de sortie d'énergie (200) est configuré pour produire de l'énergie pulsée vers le cathéter d'ablation (100), et dans lequel le contrôleur (300) comprend le support de stockage lisible (530) selon l' une quelconque des revendications 1 à 11.

13. Le système d'ablation selon la revendication 12, i) dans lequel un capteur de pression est prévu à l'extrémité distale du cathéter d'ablation (100), ce capteur de pression étant configuré pour détecter l'amplitude et la direction d'une force de contact entre l'extrémité distale du cathéter d'ablation (100) et un tissu cible,

dans lequel le contrôleur (300) est en outre configuré pour : déterminer un angle d'ajustement établi entre l'extrémité distale du cathéter d'ablation (100) et le tissu cible en fonction de la direction de la force de contact entre l'extrémité distale du cathéter d'ablation (100) et le tissu cible détectée par le capteur de pression ; et

dans lequel le système d'ablation comprend en outre un dispositif d'affichage (400) connecté de manière communicative au contrôleur (300), dans lequel le dispositif d'affichage (400) est configuré pour afficher en temps réel une résistance, l'amplitude et la direction de la force de contact entre l'extrémité distale du cathéter d'ablation (100) et le tissu cible, ainsi qu'un angle d'ajustement entre l'extrémité distale du cathéter d'ablation (100) et le tissu cible ; ou

ii) dans lequel un capteur de température est en outre prévu à l'extrémité distale du cathéter d'ablation (100), le capteur de température est configuré pour détecter une température d'ablation n du tissu cible.

14. Un dispositif électronique comprenant un processeur (510) et le support de stockage lisible (530) selon l'une quelconque des revendications 1 à 13.

CONTROL ABLATION CATHETER TO APPLY PULSED ENERGY TO TARGET TISSUE AND DETERMINE ELECTRIC FIELD ENERGY AND RESISTANCE DURING ABLATION PROCESS — S100

CALCULATE THEORETICAL ABLATION LESION SIZE ACCORDING TO ELECTRIC FIELD ENERGY AND RESISTANCE — S200

NO

IS DIFFERENCE BETWEEN THEORETICAL AND TARGET ABLATION LESION SIZES WITHIN FIRST PREDETERMINED RANGE? — S300

YES

CONTROL ABLATION CATHETER TO STOP APPLYING PULSED ENERGY TO TARGET TISSUE — S400

Fig. 1

Fig. 2

Fig. 3

Fig. 4

Fig. 5

Fig. 6

Fig. 7

Fig. 8

Fig. 9

```
                    ┌─────────────────────────────────────────┐
                    │                 START                   │
                    └─────────────────────────────────────────┘
                                      │
                                      ▼
                    ┌─────────────────────────────────────────┐◄──────┐
                    │       CONFIGURE PULSE PARAMETERS         │       │
                    └─────────────────────────────────────────┘       │
                                      │                                │
                                      ▼                                │  NO
                    ┌─────────────────────────────────────────┐       │
                    │       CALCULATE PULSE DOSE Dose          │       │
                    └─────────────────────────────────────────┘       │
                                      │                                │
                                      ▼                                │
              ◄ IS Dose IN SECOND PREDETERMINED RANGE? ►───────────────┘
                                      │
                                    YES
                                      ▼
              ◄ DOES RESISTANCE EXCEED SECOND THRESHOLD? ►
                                      │
                                    YES
                                      ▼
              ◄ IS MAGNITUDE OF CONTACT FORCE GREATER
                 THAN OR EQUAL TO FIRST THRESHOLD? ►
                                      │
                                    YES
                                      ▼
                    ┌─────────────────────────────────────────┐◄──────┐
                    │             INITIATE ABLATION            │       │
                    └─────────────────────────────────────────┘       │
                                      │                                │
                                      ▼                                │  NO
                    ┌─────────────────────────────────────────┐       │
                    │         CALCULATE THEORETICAL            │       │
                    │        ABLATION LESION SIZE             │       │
                    └─────────────────────────────────────────┘       │
                                      │                                │
                                      ▼                                │
              ◄ IS DIFFERENCE BETWEEN THEORETICAL
                 AND TARGET ABLATION LESION SIZES
                 WITHIN FIRST PREDETERMINED RANGE? ►──────────────────┘
                                      │
                                    YES
                                      ▼
                    ┌─────────────────────────────────────────┐
                    │             STOP ABLATION                │
                    └─────────────────────────────────────────┘
```

Fig.10

Fig.11

Fig. 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2011066147 A1
- CN 113768616 A